(19) Europäisches Patentamt
European Patent Office
Office européen des brevets

(11) EP 2 380 888 A1

(12) **EUROPEAN PATENT APPLICATION**
published in accordance with Art. 153(4) EPC

(43) Date of publication:
**26.10.2011 Bulletin 2011/43**

(21) Application number: **10733337.9**

(22) Date of filing: **19.01.2010**

(51) Int Cl.:
***C07D 401/14*** *(2006.01)* ***C09K 11/06*** *(2006.01)*
***H01L 51/50*** *(2006.01)*

(86) International application number:
**PCT/JP2010/000243**

(87) International publication number:
**WO 2010/084729 (29.07.2010 Gazette 2010/30)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.01.2009 JP 2009011837**

(71) Applicants:
- **Hodogaya Chemical Co., Ltd.**
  **Tokyo 105-0011 (JP)**
- **Shinshu University**
  **Matsumoto-shi, Nagano 390-8621 (JP)**

(72) Inventors:
- **YOKOYAMA, Norimasa**
  **Tsukuba-shi**
  **Ibaraki 305-0841 (JP)**
- **HAYASHI, Shuichi**
  **Tsukuba-shi**
  **Ibaraki 305-0841 (JP)**
- **TANIGUCHI, Yoshio**
  **Ueda-shi**
  **Nagano 386-8567 (JP)**
- **ICHIKAWA, Musubu**
  **Ueda-shi**
  **Nagano 386-8567 (JP)**
- **MATSUKI, Shinichi**
  **Ueda-shi**
  **Nagano 386-8567 (JP)**

(74) Representative: **Müller-Boré & Partner**
**Patentanwälte**
**Grafinger Straße 2**
**81671 München (DE)**

(54) **COMPOUND HAVING TRIAZOLE RING STRUCTURE WITH PYRIDYL GROUP ATTACHED THERETO, AND ORGANIC ELECTROLUMINESCENT ELEMENT**

(57) A host compound for a light emitting layer, which is excellent in electron transporting capability and hole blocking capability, has a high triplet excitation level and is capable of completely confining triplet excitons of a phosphorescent material, is provided as a material for an organic electroluminescent device having a high efficiency, and an organic electroluminescent device having a high efficiency and a high luminance is provided by using this compound.

The compound having a triazole ring structure to which a pyridyl group substituted with a carbazolyl group is bonded, is represented by the following general formula (1), and the organic electroluminescent device containing a pair of electrodes and at least one organic layer sandwitched therebetween is **characterized in that** the compound, which is represented by the following general formula (1) and has a triazole ring structure to which a pyridyl group substituted with a carbazolyl group is bonded, is used as a constitutional material of at least one organic layer in the organic electroluminescent device.

EP 2 380 888 A1

[Chemical Formula 1]

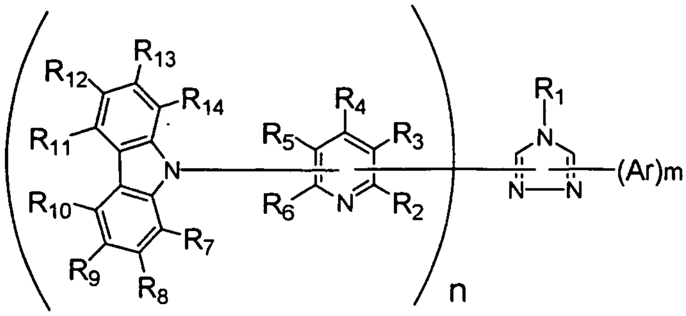

(1)

## Description

Technical Field

The present invention relates to a compound suitable for an organic electroluminescent device, a self luminescence device favorable for various kinds of display apparatuses, and to the device, and more specifically to a compound having a triazole ring structure to which a pyridyl group substituted with a carbazolyl group is bonded, and an organic electroluminescent device using the compound.

Background Art

An organic electroluminescent device is a self luminescence device, and thus is brighter and excellent in visibility as compared to a liquid crystal device, thereby enabling clear display, and therefore it has been actively studied.

As an attempt for enhancing the luminous efficiency of the device in recent years, a device that generates phosphorescence by using a phosphorescent material, i.e., utilizes luminescence from a triplet excitation state, is being developed. According to the theory of excitation state, the use of phosphorescence is expected to provide notable increase of the luminous efficiency, i.e., it may enable a luminous efficiency that is approximately four times the ordinary fluorescence.
In 1993, M.A. Baldo, et al. of Princeton University realized an external quantum efficiency of 8% with a phosphorescent device using an iridium complex.

A phosphorescent material undergoes concentration quenching, and thus is supported by doping the phosphorescent material into a charge transporting compound, which is generally referred to as a host compound. The phosphorescent material supported is referred to as a guest compound. As the host compound, 4,4'-di(N-carbazolyl) biphenyl (which is hereinafter abbreviated as CBP) represented by the following formula has been generally used (see, for example, Non-patent Document 1).

[Chemical Formula 1]

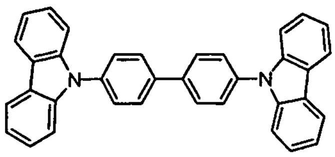

However, it has been pointed out that CBP is poor in stability in a thin film state due to the strong crystallinity thereof. Accordingly, it has not been possible to obtain device characteristics, such as high-luminance emission, that are satisfactory in situations requiring heat resistance.

Under the circumstances, 4,4',4''-tri(N-carbazolyl)triphenylamine (which is hereinafter referred to as TCTA) represented by the following formula has been proposed as a new host compound, and it has been confirmed that the compound has a luminous efficiency that is comparable to CBP (see, for example, Non-patent Document 2).

[Chemical Formula 2]

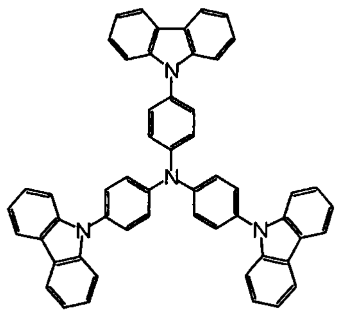

With the progress of the studies on phosphorescent devices, the energy transfer process between the phosphorescent material and the host compound is being clarified, and it has been clarified that for enhancing the luminous efficiency, the triplet excitation level of the host compound is necessarily higher than the triplet excitation level of the phosphorescent material. Accordingly, there is a need for a host compound having a higher triplet excitation level than CBP, in addition to the stability in a thin film state. Through the investigations for a host compound having a higher triplet excitation level, it has been found that an electron transporting or bipolar transporting host compound doped with an iridium complex provides a high luminous efficiency (see, for example Non-patent Document 3).

Furthermore, a green phosphorescent material $Ir(ppy)_3$ represented by the following formula:

[Chemical Formula 3]

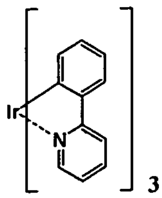

is doped into a mixed host compound containing the TCTA, which is a hole transporting host compound, and TPBI represented by the following formula:

[Chemical Formula 4]

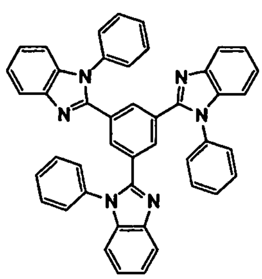

which is an electron transporting host compound, making a light emitting layer, and TCTA is used in an electron blocking layer for confining triplet excitons, whereby a high efficiency and a low voltage driving are achieved (see, for example, Non-patent Document 4).

A blue phosphorescent material FIrpic represented by the following formula:

[Chemical Formula 5]

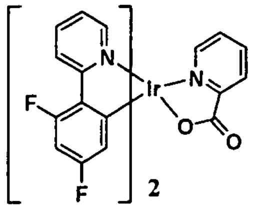

is doped into CBP as a host compound of a light emitting layer, thereby making a phosphorescent device, which exhibits an external quantum efficiency of only 6%. It has been considered that this is because while the triplet excitation level of FIrpic is 2.62 eV, the triplet excitation level of CBP is as low as 2.56 eV, and thus the confinement of triplet excitons of FIrpic is insufficient.

This has been evidenced by the fact that the photoluminescence intensity of a thin film of CBP doped with FIrpic exhibits temperature dependency (see, for example, Non-patent Document 5).

The triplet excitation level of TCTA, which is used as an electron blocking layer of the green phosphorescent device, is 2.60 eV, which is considered to be still insufficient for the confinement of triplet excitons of FIrpic.

Accordingly, for enhancing the luminous efficiency of a phosphorescent device, a host compound of a light emitting layer capable of completely confining triplet excitons of a phosphorescent material is demanded.

Related Art Documents

Patent Document

Patent Document 1: JP-A-2007-022986

Non-patent Documents

Non-patent Document 1: Appl. Phys. Let., 75, 4 (1999)
Non-patent Document 2: 9th Workshop, Molecular Electronics and Bioelectronics Group, Japan Society of Applied Physics, 17 (2001)
Non-patent Document 3: Yuki EL Display (Organic EL Display), published by Ohmsha, Ltd., 90 (2005)
Non-patent Document 4: SID07 DIGEST, 837 (2007)
Non-patent Document 5: Journal of Molecular Electronics and Bioelectronics Group, Japan Society of Applied Physics, 14(1), 23 (2003)
Non-patent Document 6: Jikken Kagaku Koza (Lectures on Experimental Chemistry), 4th Ed. , vol. 7, pp. 384 to 398, edited by The Chemical Society of Japan, published by Maruzen Co., Ltd. (1992)
Non-patent Document 7: Preprint, 1st Meeting of Symposium for Organic EL, 19 (2005)

Disclosure of the Invention

Problems to be solved by the Invention

An object of the invention is to provide, as a material for an organic electroluminescent device having a high efficiency, a compound having hole blocking property and a host compound for a light emitting layer that have a high triplet excitation level and are capable of completely confining triplet excitons of a phosphorescent material, and to provide an organic electroluminescent device having a high efficiency and a high luminance using the compound. The physical characteristics that are owned by the organic compound to be provided by the invention include (1) a high triplet

excitation level, (2) a bipolar transporting property, and (3) stability in a thin film state, and the compound may be used as a constitutional material of an electron transport layer, a hole blocking layer or a light emitting layer. The physical characteristics that are owned by the organic electroluminescent device to be provided by the invention include (1) a high luminous efficiency, (2) a high emission luminance, and (3) a low practical driving voltage.

Means for solving the Problems

For achieving the aforementioned object, the present inventors have designed and chemically synthesized a compound with triplet excitation level as an index in view of the facts that a triazole ring structure to which a pyridyl group is bonded, has an electron transporting capability, and a carbazole structure has a hole transporting capability, and the present inventors have actually measured the triplet excitation level of the compound, thereby finding a novel compound having characteristics suitable for a phosphorescent device, the compound having a triazole ring structure to which a pyridyl group substituted with a carbazolyl group is bonded. The inventors have also produced various organic electroluminescent devices using the compound and evaluated the characteristics of the devices, thereby achieving the invention.

The invention relates to a compound of the following general formula (1) having a triazole ring structure to which a pyridyl group substituted with a carbazolyl group is bonded, and relates to an organic electroluminescent device containing a pair of electrodes and at least one organic layer sandwitched therebetween, at least one organic layer containing, as a constitutional material, a compound of the following general formula (1) having a triazole ring structure to which a pyridyl group substituted with a carbazolyl group is bonded.

[Chemical Formula 6]

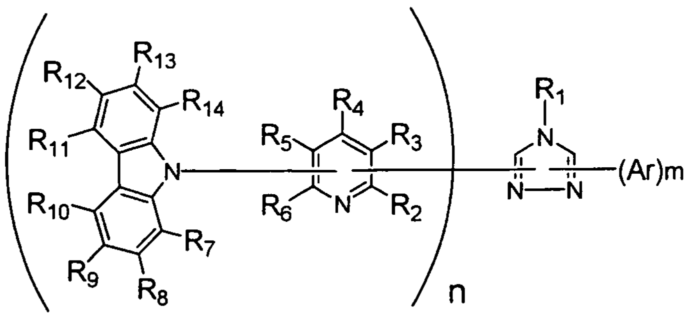

(1)

In the general formula (1), wherein Ar represents a substituted or unsubstituted aromatic hydrocarbon group, a substituted or unsubstituted aromatic heterocyclic group or a substituted or unsubstituted condensed polycyclic aromatic group; $R_1$ represents a substituted or unsubstituted aromatic hydrocarbon group, a substituted or unsubstituted aromatic heterocyclic group or a substituted or unsubstituted condensed polycyclic aromatic group; two of $R_2$, $R_3$, $R_4$, $R_5$ and $R_6$ represent a bonding group to a triazole ring or a carbazolyl group, and the others may be the same or different from each other, each representing a hydrogen atom, a deuterium atom, a fluorine atom, a cyano group, a linear or branched alkyl group having from 1 to 6 carbon atoms, a substituted or unsubstituted aromatic hydrocarbon group, a substituted or unsubstituted aromatic heterocyclic group or a substituted or unsubstituted condensed polycyclic aromatic group ; $R_7$, $R_8$, $R_9$, $R_{10}$, $R_{11}$, $R_{12}$, $R_{13}$ and $R_{14}$ may be the same or different from each other, each representing a hydrogen atom, a deuterium atom, a fluorine atom, a cyano group, a linear or branched alkyl group having from 1 to 6 carbon atoms, a substituted or unsubstituted aromatic hydrocarbon group, a substituted or unsubstituted aromatic heterocyclic group or a substituted or unsubstituted condensed polycyclic aromatic group; m represents 0 or 1; and n represents an integer of 1 or 2, provided that a sum of m and n is 2.

Examples of the aromatic hydrocarbon group, the aromatic heterocyclic group or the condensed polycyclic aromatic group in the substituted or unsubstituted aromatic hydrocarbon group, the substituted or unsubstituted aromatic heterocyclic group or the substituted or unsubstituted condensed polycyclic aromatic group represented by Ar in the general formula (1) include a phenyl group, a biphenylyl group, a terphenylyl group, a tetrakisphenyl group, a styryl group, a naphthyl group, an anthryl group, an acenaphthenyl group, a fluorenyl group, a phenanthryl group, an indenyl group, a pyrenyl group, a pyridyl group, a pyrimidyl group, a furanyl group, a pyronyl group, a thienyl group, a quinolyl

group, a benzofuranyl group, a benzothienyl group, an indolyl group, a carbazolyl group, a benzoxazolyl group, a quinoxalyl group, a benzoimidazolyl group, a pyrazolyl group, a dibenzofuranyl group and a dibenzothienyl group.

Examples of the substituent in the substituted aromatic hydrocarbon group, the substituted aromatic heterocyclic group or the substituted condensed polycyclic aromatic group represented by Ar in the general formula (1) include a deuterium atom, a fluorine atom, a chlorine atom, a cyano group, a hydroxyl group, a nitro group, a linear or branched alkyl group having from 1 to 6 carbon atoms, an alkoxy group, an amino group, a trif luoromethyl group, a phenyl group, a biphenylyl group, a terphenylyl group, a naphthyl group, a phenanthryl group, an aralkyl group, a fluorenyl group, an indenyl group, a pyridyl group, a pyrimidyl group, a furanyl group, a pyronyl group, a thienyl group, a quinolyl group, a benzofuranyl group, a benzothienyl group, an indolyl group, a carbazolyl group, a carbollyl group, a benzoxazolyl group, a quinoxalyl group, a benzoimidazolyl group, a pyrazolyl group, a dibenzofuranyl group and a dibenzothienyl group, and the substituent may be further substituted.

Examples of the aromatic hydrocarbon group, the aromatic heterocyclic group or the condensed polycyclic aromatic group in the substituted or unsubstituted aromatic hydrocarbon group, the substituted or unsubstituted aromatic heterocyclic group or the substituted or unsubstituted condensed polycyclic aromatic group represented by $R_1$ in the general formula (1) as the substituent on the triazole group include a phenyl group, a biphenylyl group, a terphenylyl group, a tetrakisphenyl group, a styryl group, a naphthyl group, an anthryl group, an acenaphthenyl group, a fluorenyl group, a phenanthryl group, an indenyl group, a pyrenyl group, a pyrimidyl group, a furanyl group, a pyronyl group, a thienyl group, a quinolyl group, a benzofuranyl group, a benzothienyl group, an indolyl group, a carbazolyl group, a carbollyl group, a benzoxazolyl group, a quinoxalyl group, a benzoimidazolyl group, a pyrazolyl group, a dibenzofuranyl group and a dibenzothienyl group.

Examples of the substituent in the substituted aromatic hydrocarbon group, the substituted aromatic heterocyclic group or the substituted condensed polycyclic aromatic group represented by $R_1$ in the general formula (1) as the substituent on the triazole group include a deuterium atom, a fluorine atom, a chlorine atom, a cyano group, a hydroxyl group, a nitro group, a linear or branched alkyl group having from 1 to 6 carbon atoms, an alkoxy group, an amino group, a trifluoromethyl group, a phenyl group, a biphenylyl group, a terphenylyl group, a naphthyl group, a phenanthryl group, an aralkyl group, a fluorenyl group, an indenyl group, a pyridyl group, a pyrimidyl group, a furanyl group, a pyronyl group, a thienyl group, a quinolyl group, a benzofuranyl group, a benzothienyl group, an indolyl group, a carbazolyl group, a carbollyl group, a benzoxazolyl group, a quinoxalyl group, a benzoimidazolyl group, a pyrazolyl group, a dibenzofuranyl group and a dibenzothienyl group, and the substituent may be further substituted.

Examples of the aromatic hydrocarbon group, the aromatic heterocyclic group or the condensed polycyclic aromatic group in the substituted or unsubstituted aromatic hydrocarbon group, the substituted or unsubstituted aromatic heterocyclic group or the substituted or unsubstituted condensed polycyclic aromatic group represented byre to $R_6$ in the general formula (1) as the substituent on the pyridyl group include a phenyl group, a biphenylyl group, a terphenylyl group, a tetrakisphenyl group, a styryl group, a naphthyl group, an anthryl group, an acenaphthenyl group, a fluorenyl group, a phenanthryl group, an indenyl group, a pyrenyl group, a pyrimidyl group, a furanyl group, a pyronyl group, a thienyl group, a quinolyl group, a benzofuranyl group, a benzothienyl group, an indolyl group, a carbazolyl group, a carbollyl group, a benzoxazolyl group, a quinoxalyl group, a benzoimidazolyl group, a pyrazolyl group, a dibenzofuranyl group and a dibenzothienyl group.

Examples of the substituent in the substituted aromatic hydrocarbon group, the substituted aromatic heterocyclic group or the substituted condensed polycyclic aromatic group represented by $R_2$ to $R_6$ in the general formula (1) as the substituent on the pyridyl group include a deuterium atom, a fluorine atom, a chlorine atom, a cyano group, a hydroxyl group, a nitro group, a linear or branched alkyl group having from 1 to 6 carbon atoms, an alkoxy group, an amino group, a trifluoromethyl group, a phenyl group, a biphenyl group, a terphenyl group, a naphthyl group, a phenanthryl group, an aralkyl group, a fluorenyl group, an indenyl group, a pyridyl group, a pyrimidyl group, a furanyl group, a pyronyl group, a thienyl group, a quinolyl group, a benzofuranyl group, a benzothienyl group, an indolyl group, a carbazolyl group, a carbollyl group, a benzoxazolyl group, a quinoxalyl group, a benzoimidazolyl group, a pyrazolyl group, a dibenzofuranyl group and a dibenzothienyl group, and the substituent may be further substituted.

Examples of the aromatic hydrocarbon group, the aromatic heterocyclic group or the condensed polycyclic aromatic group in the substituted or unsubstituted aromatic hydrocarbon group, the substituted or unsubstituted aromatic heterocyclic group or the substituted or unsubstituted condensed polycyclic aromatic group represented by $R_7$ to $R_{14}$ in the general formula (1) as the substituent on the carbazolyl group include a phenyl group, a biphenylyl group, a terphenylyl group, a tetrakisphenyl group, a styryl group, a naphthyl group, an anthryl group, an acenaphthenyl group, a fluorenyl group, a phenanthryl group, an indenyl group, a pyrenyl group, a pyrimidyl group, a furanyl group, a pyronyl group, a thienyl group, a quinolyl group, a benzofuranyl group, a benzothienyl group, an indolyl group, a carbazolyl group, a benzoxazolyl group, a quinoxalyl group, a benzoimidazolyl group, a pyrazolyl group, a dibenzofuranyl group and a dibenzothienyl group.

Examples of the substituent in the substituted aromatic hydrocarbon group, the substituted aromatic heterocyclic group or the substituted condensed polycyclic aromatic group represented by $R_7$ to $R_{14}$ in the general formula (1) as

the substituent on the carbazol group include a deuterium atom, a fluorine atom, a chlorine atom, a cyano group, a hydroxyl group, a nitro group, a linear or branched alkyl group having from 1 to 6 carbon atoms, an alkoxy group, an amino group, a trifluoromethyl group, a phenyl group, a biphenylyl group, a terphenylyl group, a naphthyl group, a phenanthryl group, an aralkyl group, a fluorenyl group, an indenyl group, a pyridyl group, a pyrimidyl group, a furanyl group, a pyronyl group, a thienyl group, a quinolyl group, a benzofuranyl group, a benzothienyl group, an indolyl group, a carbazolyl group, a carbollyl group, a benzoxazolyl group, a quinoxalyl group, a benzoimidazolyl group, a pyrazolyl group, a dibenzofuranyl group and a dibenzothienyl group, and the substituent may be further substituted.

The compound of the general formula (1) of the invention having a triazole ring structure to which a pyridyl group substituted with a carbazolyl group is bonded, is a novel compound having a higher triplet excitation level than a conventional material for a hole blocking layer, an excellent capability of confining triplet excitons, and stability in a thin film state.

The compound of the general formula (1) of the invention having a triazole ring structure to which a pyridyl group substituted with a carbazolyl group is bonded, may be used as a constitutional material for an electron transport layer, a hole blocking layer or a light emitting layer of an organic electroluminescent device (which is hereinafter abbreviated as an organic EL device). The use of the compound of the invention, which is excellent in bipolar transporting capability as compared to the conventional materials, achieves functions of enhancing the luminous efficiency and lowering the practical driving voltage.

Advantages of the Invention

The compound of the invention having a triazole ring structure to which a pyridyl group substituted with a carbazolyl group is bonded, is useful as a compound for an electron transport layer, a compound for a hole blocking layer or a host compound for a light emitting layer of an organic EL device, and the production of an organic EL device using the compound provides an organic EL device having a high efficiency, a high luminance and a low driving voltage.

Brief Description of the Drawings

[Fig. 1] The figure is an illustration showing a $^1$H-NMR spectrum chart of the compound of Example 1 (Compound 8) of the invention.

[Fig. 2] The figure is an illustration showing a structure of an EL device in Example 5 and Comparative Example 1.

Embodiments for carrying out the Invention

The compound of the invention having a triazole ring structure to which a pyridyl group substituted with a carbazolyl group is bonded, is a novel compound. The compound may be synthesized, for example, by synthesizing a halogenopyridyltriazole intermediate through cyclization reaction of a corresponding acylhydrazine with an arylamine in the presence of phosphorus trichloride, and then synthesizing the compound having a triazole ring structure to which a pyridyl group substituted with a carbazolyl group is bonded, through an Ullmann reaction or amination reaction using a palladium catalyst with corresponding carbazoles.

Preferred examples of the compound of the general formula (1) of the invention having a triazole ring structure to which a pyridyl group substituted with a carbazolyl group is bonded, are shown below, but the invention is not limited to the compounds.

[Chemical Formula 7]

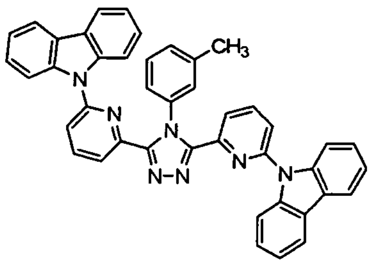

(Compound 7)

[Chemical Formula 8]

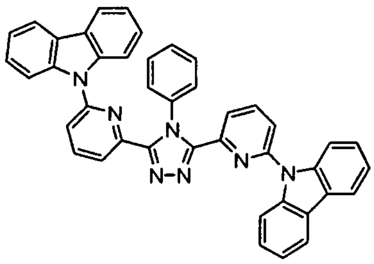

(Compound 8)

[Chemical Formula 9]

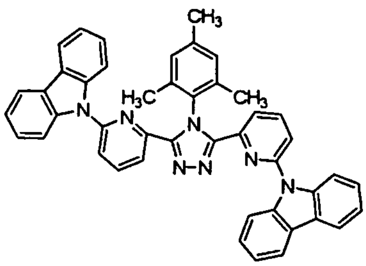

(Compound 9)

[Chemical Formula 10]

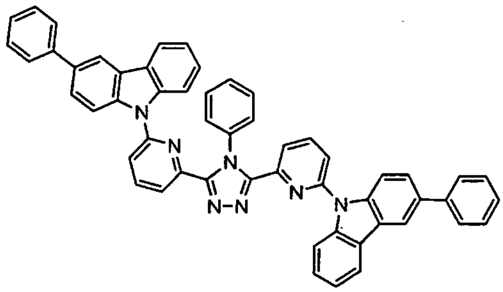

(Compound 10)

[Chemical Formula 11]

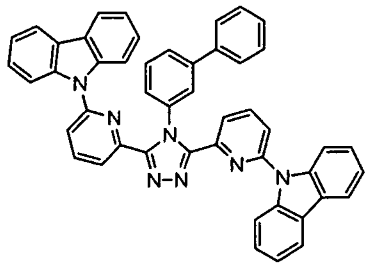

(Compound 11)

[Chemical Formula 12]

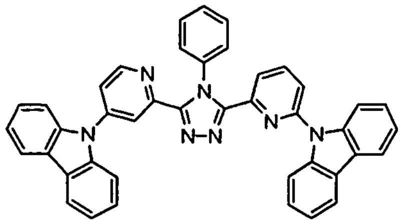

(Compound 12)

[Chemical Formula 13]

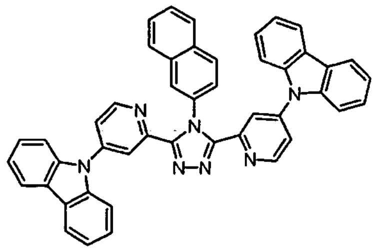

(Compound 13)

[Chemical Formula 14]

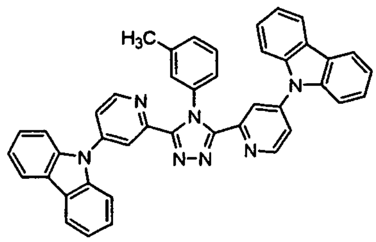

(Compound 14)

[Chemical Formula 15]

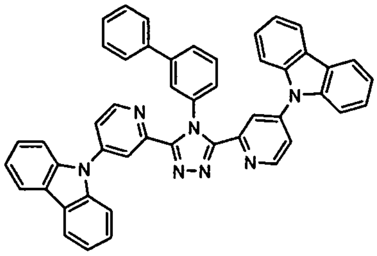

(Compound 15)

[Chemical Formula 16]

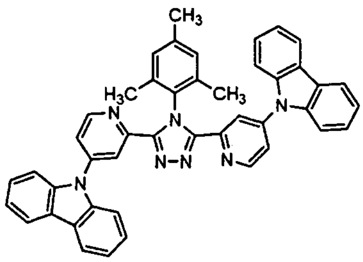

(Compound 16)

[Chemical Formula 17]

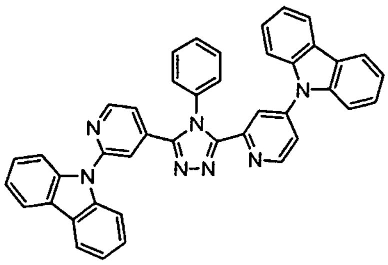

(Compound 17)

[Chemical Formula 18]

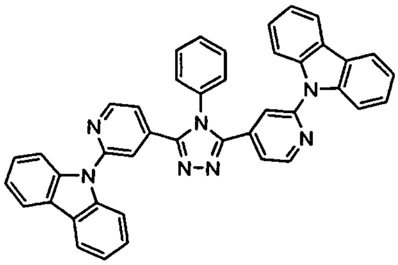

(Compound 18)

[Chemical Formula 19]

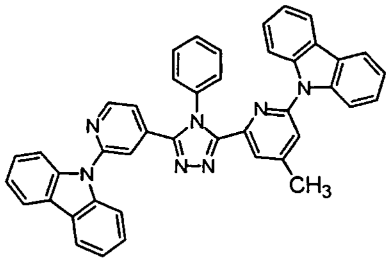

(Compound 19)

[Chemical Formula 20]

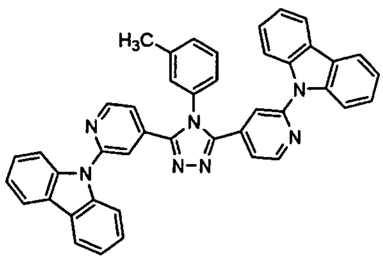

(Compound 20)

[Chemical Formula 21]

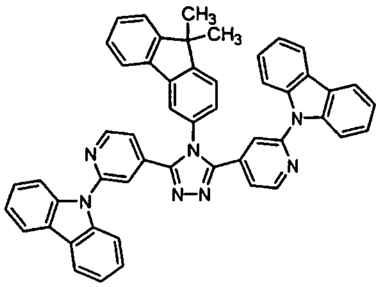

(Compound 21)

[Chemical Formula 22]

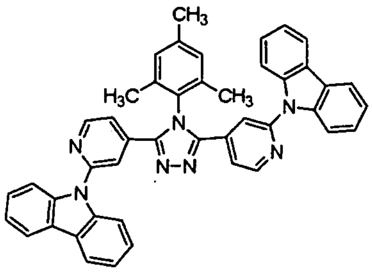

(Compound 22)

[Chemical Formula 23]

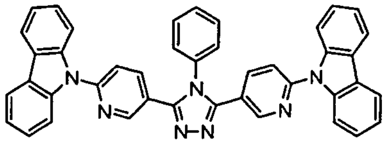

(Compound 23)

[Chemical Formula 24]

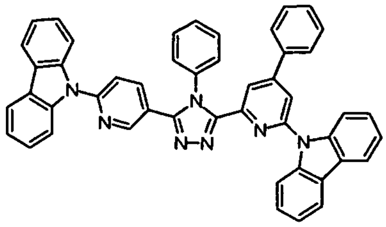

(Compound 24)

[Chemical Formula 25]

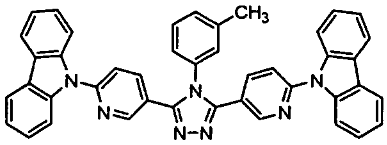

(Compound 25)

[Chemical Formula 26]

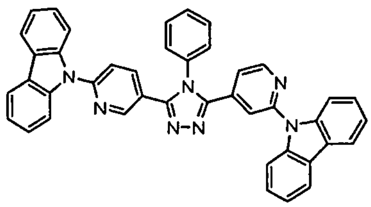

(Compound 26)

[Chemical Formula 27]

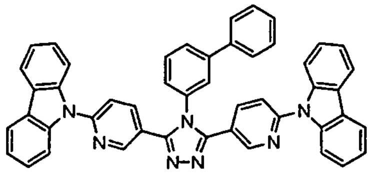

(Compound 27)

[Chemical Formula 28]

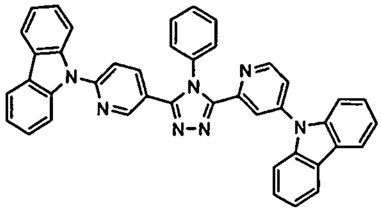

(Compound 28)

[Chemical Formula 29]

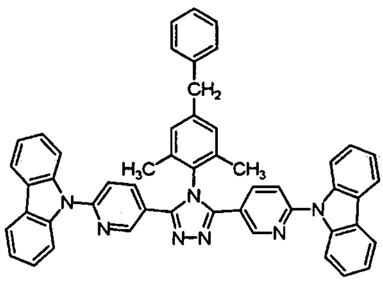

(Compound 29)

[Chemical Formula 30]

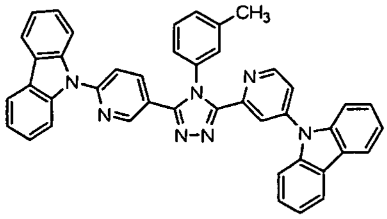

(Compound 30)

[Chemical Formula 31]

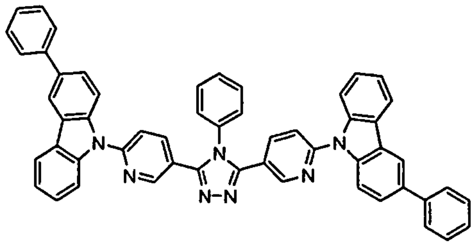

(Compound 31)

[Chemical Formula 32]

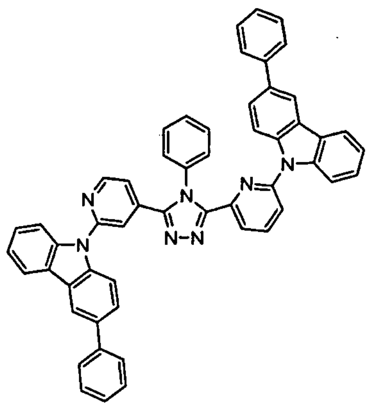

(Compound 32)

[Chemical Formula 33]

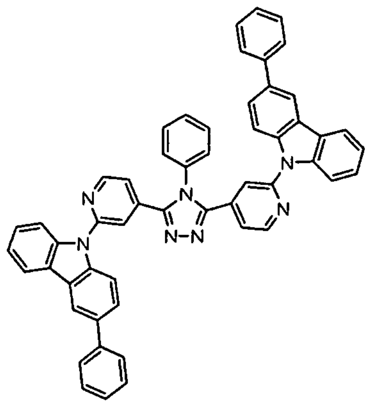

(Compound 33)

[Chemical Formula 34]

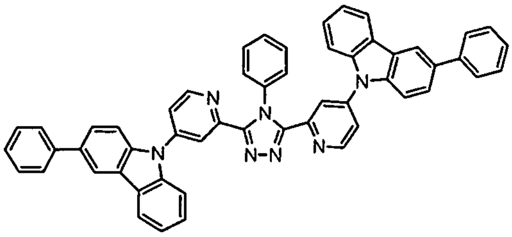

(Compound 34)

[Chemical Formula 35]

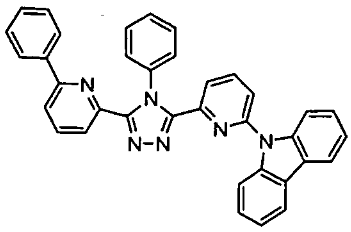

(Compound 35)

[Chemical Formula 36]

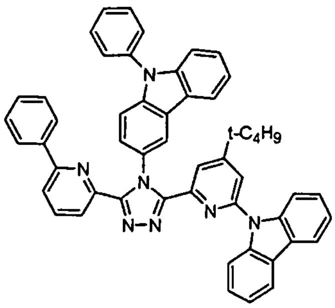

(Compound 36)

[Chemical Formula 37]

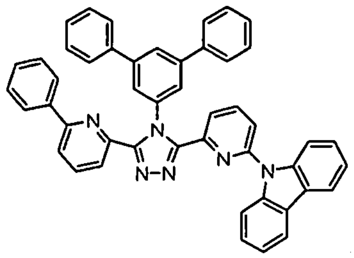

(Compound 37)

[Chemical Formula 38]

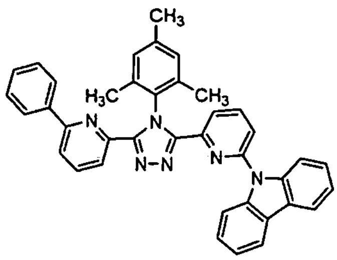

(Compound 38)

[Chemical Formula 39]

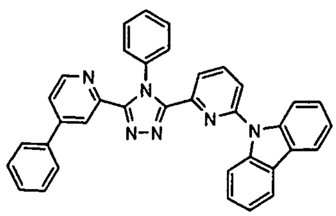

(Compound 39)

[Chemical Formula 40]

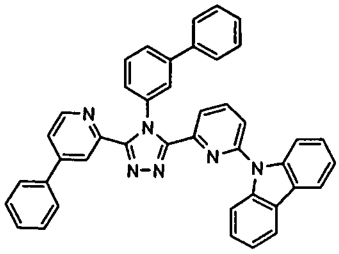

(Compound 40)

[Chemical Formula 41]

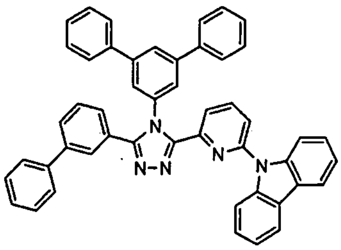

(Compound 41)

[Chemical Formula 42]

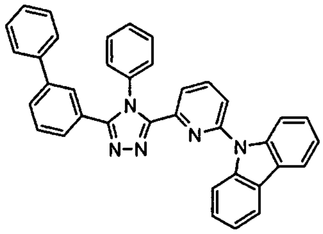

(Compound 42)

[Chemical Formula 43]

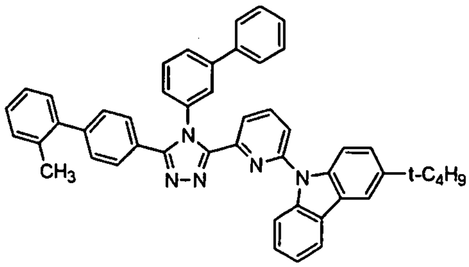

(Compound 43)

[Chemical Formula 44]

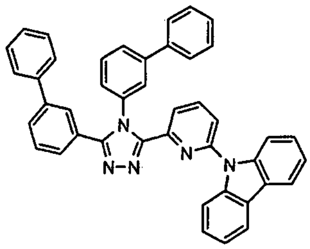

(Compound 44)

[Chemical Formula 45]

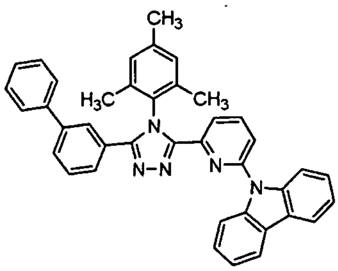

(Compound 45)

[Chemical Formula 46]

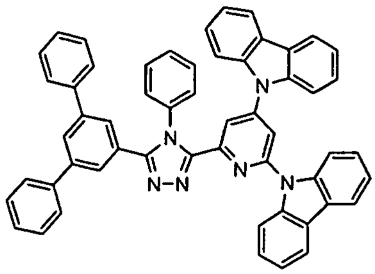

(Compound 46)

[Chemical Formula 47]

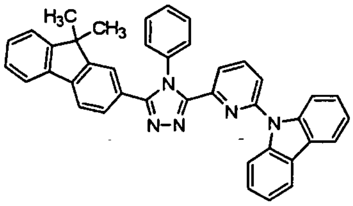

(Compound 47)

[Chemical Formula 48]

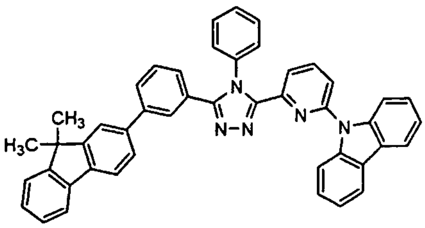

(Compound 48)

[Chemical Formula 49]

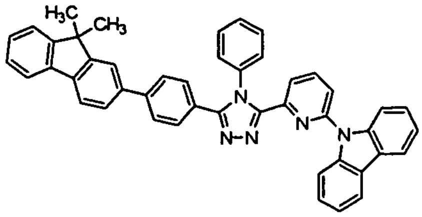

(Compound 49)

[Chemical Formula 50]

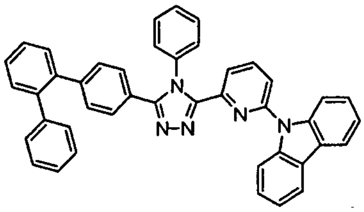

(Compound 50)

[Chemical Formula 51]

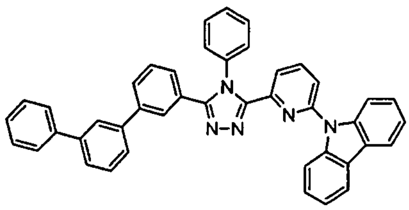

(Compound 51)

[Chemical Formula 52]

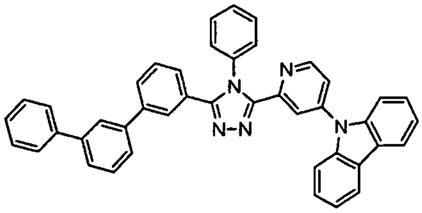

(Compound 52)

[Chemical Formula 53]

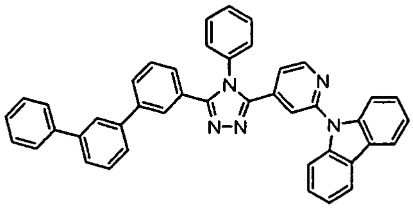

(Compound 53)

[Chemical Formula 54]

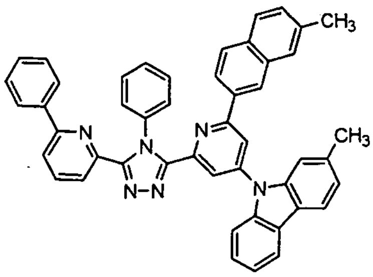

(Compound 54)

[Chemical Formula 55]

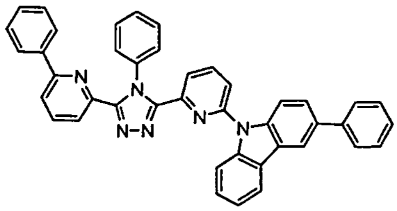

(Compound 55)

[Chemical Formula 56]

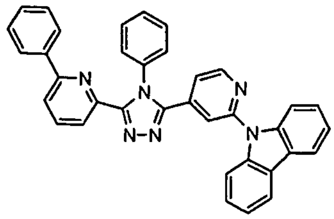

(Compound 56)

[Chemical Formula 57]

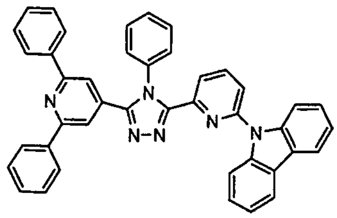

(Compound 57)

[Chemical Formula 58]

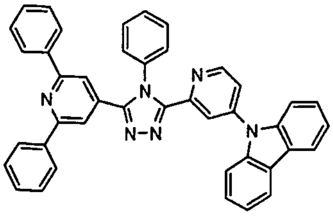

(Compound 58)

[Chemical Formula 59]

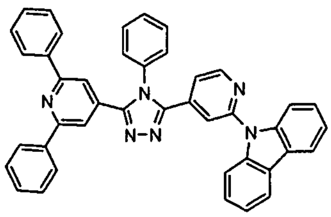

(Compound 59)

[Chemical Formula 60]

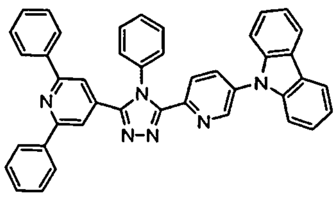

(Compound 60)

[Chemical Formula 61]

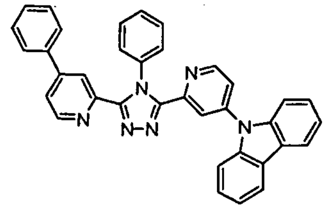

(Compound 61)

[Chemical Formula 62]

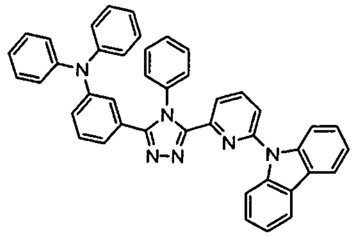

(Compound 62)

[Chemical Formula 63]

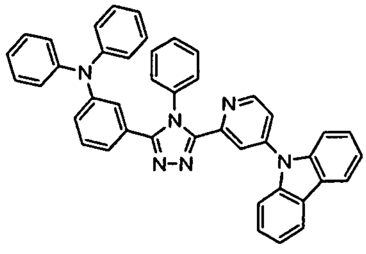

(Compound 63)

[Chemical Formula 64]

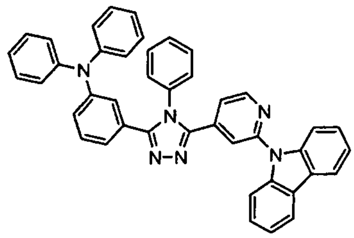

(Compound 64)

[Chemical Formula 65]

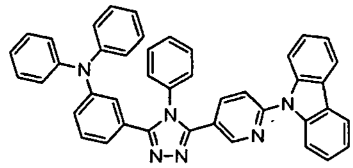

(Compound 65)

[Chemical Formula 66]

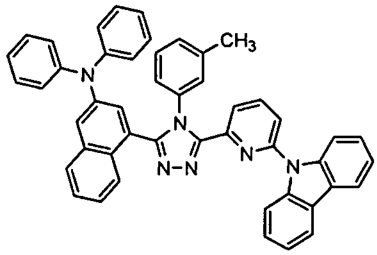

(Compound 66)

[Chemical Formula 67]

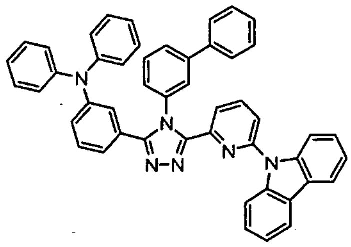

(Compound 67)

[Chemical Formula 68]

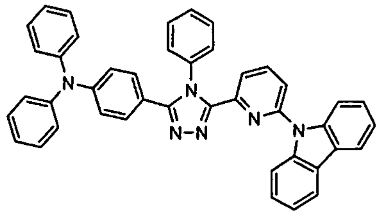

(Compound 68)

[Chemical Formula 69]

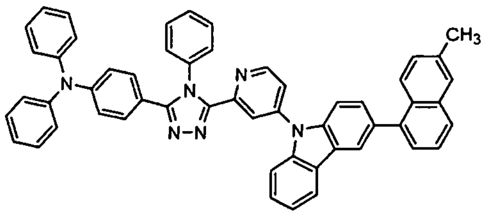

(Compound 69)

[Chemical Formula 70]

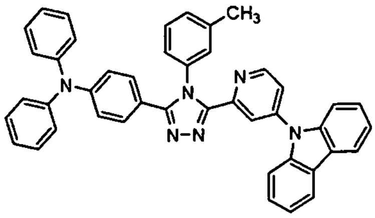

(Compound 70)

[Chemical Formula 71]

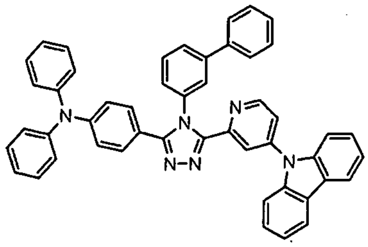

(Compound 71)

[Chemical Formula 72]

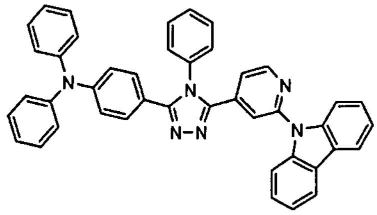

(Compound 72)

[Chemical Formula 73]

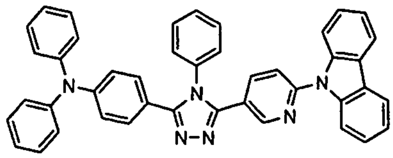

(Compound 73)

[Chemical Formula 74]

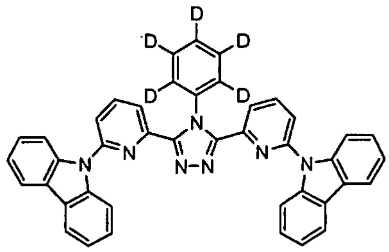

(Compound 74)

[Chemical Formula 75]

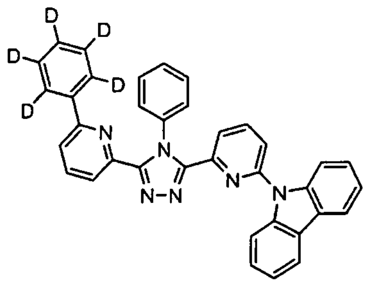

(Compound 75)

[Chemical Formula 76]

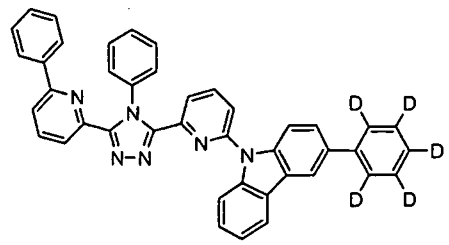

(Compound 76)

The compounds were purified through purification by column chromatography, adsorption purification with activated carbon, activated clay or the like, recrystallization or crystallization from a solvent, or the like. The compounds were identified by NMR analysis. As physical properties, DSC measurement (Tg) and measurement of the melting point were performed. The melting point may be an index of the vapor deposition property, and the glass transition point (Tg) may be an index of the stability in a thin film state.

The melting point and the glass transition point were measured by using powder of the compound with a high-sensitivity differential scanning calorimeter, DSC3100S, produced by Bruker AXS.

The work function was measured by producing a thin film of 100 nm on an ITO substrate and measuring it with an atmospheric photoelectron spectrometer, Model AC-3, produced by Riken Keiki Co., Ltd. The work function may be an index of the hole transporting capability and the hole blocking capability.

The triplet excitation energy level of the compound of the invention can be calculated from the measured phosphorescence spectrum. The phosphorescence spectrum may be measured with a commercially available spectrophotometer. An ordinary measuring method of the phosphorescence spectrum includes a method of measuring by dissolving the compound in a solvent and irradiating with low temperature excitation light (see, for example, Non-patent Document 6), a method of vapor-depositing on a silicon substrate to form a thin film, which is the irradiated with low temperature excitation light to measure the phosphorescence spectrum (see, for example, Patent Document 1), or the like. The triplet excitation level can be calculated in such a manner that the wavelength of the first peak on the short wavelength side of the phosphorescence spectrum or the wavelength at the rise on the short wavelength side thereof is measured and then converted to the light energy value according to the following equation. The triplet excitation level may be an index of the confinement of triplet excitons of the phosphorescent material.

[Formula 1]

$$E\ (eV) = hc/\lambda$$

wherein E represents the light energy value; h represents the Planck constant ($6.63 \times 10^{-34}$ Js), c represents the velocity of light ($3.00 \times 10^{8}$ m/s), and $\lambda$ represents the wavelength (nm) at the rise on the short wavelength side of the phosphorescence spectrum. Herein, 1 eV corresponds to $1.60 \times 10^{-19}$ J.

Examples of the structure of the organic EL device of the invention include one containing a substrate having thereon in this order an anode, a hole injection layer, a hole transport layer, an electron blocking layer, a light emitting layer, a hole blocking layer, an electron transport layer and a cathode, and one further having an electron injection layer between the electron transport layer and the cathode. In the multi-layer structure, some layers may be omitted from the organic layers, and for example, the organic EL device may be structured to include an anode, a hole transport layer, a light emitting layer, an electron transport layer and a cathode, sequentially formed on a substrate.

The light emitting layer, the hole transport layer and the electron transport layer each may have a structure containing two or more layers laminated.

The hole injection layer or the hole transport layer may contain a material, which has been ordinarily used therein, p-doped with trisbromophenylamine hexachloroantimony, a polymer compound having as a partial structure thereof a structure of N,N'-diphenyl-N,N'-di(m-tolyl)benzidine (which is abbreviated as TPD), and the like.

The electron injection layer or the electron transport layer may contain a material, which has been ordinarily used therein, n-doped with a metal, such as cesium.

The anode used in the organic EL device of the invention may contain an electrode material having a large work function, such as ITO and gold. The hole injection layer may contain copper phthalocyanine, and may also contain a naphthalenediamine derivative and a starburst type triphenylamine derivative, or a coating type material. The hole transport layer of the invention may contain a compound containing a m-carbazolylphenyl group, and may also contain TPD, N,N'-diphenyl-N,N'-di(a-naphthyl)benzidine (which is abbreviated as NPD), bis[N,N-di(p-tolyl)-4-aminophenyl]cyclohexane (which is abbreviated as TPAC), or the like.

The electron blocking layer of the organic EL device of the invention may contain a compound having an electron blocking capability, for example, a carbazole derivative, such as TCTA, 9,9-bis[4-(carbazol-9-yl)phenyl]fluorene and 1,3-bis(carbazol-9-yl)benzene (which is hereinafter abbreviated as mCP), and a compound having a triphenylsilyl group and a triarylamine structure, represented by 9-[4-(carbazol-9-yl)phenyl]-9-[4-(triphenylsilyl)phenyl]-9H-fluorene.

The light emitting layer of the organic EL device of the invention is produced by doping a luminescent material referred to as a guest material into a host material having a hole injection and transporting capability. The compound of the general formula (1) of the invention having a triazole ring structure to which a pyridyl group substituted with a carbazolyl group is bonded, and TPBI exhibiting an electron transporting capability, may be used as the host material of the light emitting layer.

The guest material used in the light emitting layer of the organic EL device of the invention may be either a fluorescent material or a phosphorescent material. Examples of the fluorescent material used include such fluorescent materials as a rubrene derivative, an anthracene derivative and a coumarin derivative. Examples of the phosphorescent material include a green phosphorescent material, such as an iridium complex of phenylpyridine Ir $(ppy)_3$, a blue phosphorescent material, such as FIrpic and FIr6, and a red phosphorescent material, such as Btp2Ir(acac).

The guest material as a phosphorescent material undergoes concentration quenching, and thus is preferably doped through co-vapor-deposition in a range of from 1 to 30% by weight based on the total light emitting layer.

Furthermore, a device of a structure in which a light emitting layer produced by using a compound of a different work function as a host material is adjacently laminated on a light emitting layer produced with the compound of the present invention may be produced (see, for example, Non-patent Document 7).

The hole blocking layer of the organic EL device of the invention may contain a compound having a hole blocking capability, for example, the compound of the general formula (1) of the invention having a triazole ring structure to which a pyridyl group substituted with a carbazolyl group is bonded, a phenanthroline derivative, such as BCP, aluminum(III) bis(2-methyl-8-quinolinate)-4-phenylphenolate (which is hereinafter abbreviated as BAlq), an oxazole derivative and a triazole derivative.

The electron transport layer of the organic EL device of the invention may contain the compound of the general formula (1) of the invention having a triazole ring structure to which a pyridyl group substituted with a carbazolyl group is bonded, an oxadiazole derivative, a triazole derivative, tris(8-hydroxyquinoline)aluminum (which is hereinafter abbreviated as Alq) as an aluminum complex of quinoline, BAlq, or the like. Examples of the electron injection layer of the organic EL device of the invention include lithium fluoride, and it may be omitted when the electron transport layer and the cathode are appropriately selected. The cathode used in the organic EL device of the invention may contain an electrode material having a low work function, such as aluminum and an alloy of magnesium and silver.

The embodiments of the invention will be described more specifically with reference to examples below, but the invention is not limited to the examples unless they exceed the substance of the invention.

Example 1

Synthesis of 3,5-bis[6-(carbazol-9-yl)-pyridin-2-yl]-4-phenyl-1,2,4-triazole (Compound 8)

14.6 mL of aniline and 180 mL of 1,2-dichlorobenzene having been dehydrated were placed in a reaction vessel having been substituted with nitrogen, to which 2.87 mL of phosphorus trichloride was dropped at room temperature, and then the mixture was heated to 100°C, followed by stirring for 2 hours. After cooling to 50°C or less, 11.0 g of 6-bromopyridine-2-carboxylic acid-N'-(6-bromopyridine-2-carbonyl)hydrazide was added thereto, and the mixture was heated to 165°C, followed by stirring for 7 hours. After cooling the reaction solution to 50°C, water was added thereto, followed by further stirring for 1 hour. The reaction solution was extracted with chloroform, and the organic layer was washed with a potassium carbonate aqueous solution, then dried over magnesium sulfate, and concentrated under reduced pressure. The resulting solid matter was purified by column chromatography (carrier: silica gel, eluant: chloroform/hexane = 3/7 (v/v)) to provide 2.9 g (yield: 23%) of 3,5-bis(6-bromopyridin-2-yl)-4-phenyl-1,2,4-triazole as white

powder.

2.9 g of 3,5-bis(6-bromopyridin-2-yl)-4-phenyl-1,2,4-triazole obtained above, 2.3 g of carbazole, 0.2 g of copper powder, 2.6 g of potassium carbonate, 0.2 mL of dimethylsulfoxide and 25 mL of 1, 2-dichlorobenzene were placed in a reaction vessel having been substituted with argon, and heated and stirred at 150°C for 7 hours. After cooling to room temperature, 200 mL of chloroform was added thereto, the insoluble matters were removed by filtration, and the filtrate was concentrated under reduced pressure to provide a crude product. The crude product was purified by column chromatography (carrier: NH silica gel, eluant: toluene/ethyl acetate = 5/1 (v/v)) to provide 1.3 g (yield: 32%) of 3,5-bis [6-(carbazol-9-yl)-pyridin-2-yl]-4-phenyl-1,2,4-triazole (Compound 8) as white powder.

The structure of the resulting white powder was identified by NMR. The result of the $^1$H-NMR measurement is shown in Fig. 1.

In the $^1$H-NMR (DMSO-d6) spectrum, the following 27 hydrogen signals were detected : δ (ppm) = 8.26 (2H), 8.16 (4H), 8.03 (2H), 7.84 (2H), 7.45 (2H), 7.23-7.32 (15H).

Example 2

The compound of the invention was measured for a melting point and a glass transition point with a high-sensitivity differential scanning calorimeter (DSC3100S, produced by Bruker AXS).

| | Melting point | Glass transition point |
|---|---|---|
| Compound of Example 1 of Invention | 313°C | 117°C |

The compound of the invention exhibits a glass transition point of 100°C or more and is stable in a thin film state.

Example 3

A vapor-deposited film having a thickness of 100 nm was produced with the compound of the invention on an ITO substrate, and measured for a work function with an atmospheric photoelectron spectrometer (Model AC-3, produced by Riken Keiki Co., Ltd.).

| | Work function |
|---|---|
| Compound of Example 1 of Invention | 5.75 eV |
| CBP | 6.00 eV |

The compound of the invention has a favorable energy level as compared to CBP, which is ordinarily used as a host compound of a light emitting layer.

Example 4

A $1.0 \times 10^{-5}$ mol/L 2-methyltetrahydrofuran solution of the compound of the invention was prepared. The solution thus prepared was placed in a dedicated quartz tube, the oxygen content was removed by feeding pure nitrogen, and the tube was plugged with septum rubber for preventing oxygen from entering. After cooling to 77 K, a phosphorescent spectrum was measured by irradiating with excitation light with a fluorescence and phosphorescence spectrophotometer (Model FluoroMax-4, produced by Horiba, Ltd.). The wavelength at the rise on the short wavelength side of the phosphorescent spectrum was read, and the value of this wavelength was converted to the light energy for calculating the triplet excitation level.

| | Triplet excitation level |
|---|---|
| Compound of Example 1 of Invention | 3.02 eV |
| CBP | 2.56 eV |
| FIrpic | 2.62 eV |

The compound of the invention has a larger triplet energy than FIrpic and CBP, which are blue phosphorescent materials ordinarily used, and has a capability of sufficiently confining the triplet energy excited in the light emitting layer.

Example 5

An organic EL device was produced in such a manner as shown in Fig. 2 that on a glass substrate 1 having an ITO electrode as a transparent electrode 2 formed in advance, a hole transport layer 3, an electron blocking layer 4, a light emitting layer 5, an electron transport layer 6, an electron injecting layer 7 and a cathode (aluminum electrode) 8 were vapor-deposited in this order. The glass substrate 1 having ITO having a thickness of 150 nm formed thereon was rinsed with an organic solvent, and the surface thereof was cleaned by an oxygen plasma treatment. Thereafter, the glass substrate having an ITO electrode was placed in a vacuum vapor deposition device, which was depressurized to 0.001 Pa or less.

Subsequently, a film of NPD was formed to a thickness of 40 nm at a vapor deposition rate of 1.0 Å/sec as the hole transport layer 3 covering the transparent electrode 2. On the hole transport layer 3, a film of mCP was formed to a thickness of 10 nm at a vapor deposition rate of 1.0 Å/sec as the electron blocking layer 4. On the electron blocking layer 4, the compound of Example 1 of the invention (Compound 8) and FIrpic as a blue phosphorescent material were formed as the light emitting layer 5 in a thickness of 20 nm by the dual vapor deposition performed at a vapor deposition rate ratio of (compound of Example 1 of the invention (Compound 8))/(FIrpic) = 91/9. On the light emitting layer 5, a film of TPBI was formed to a thickness of 45 nm at a vapor deposition rate of 1.0 Å/sec as the electron transport layer 6. On the electron transport layer 6, a film of lithium fluoride was formed to a thickness of 0. 5 nm at a vapor deposition rate of 0.1 Å/sec as the electron injection layer 7. Finally, aluminum was vapor-deposited to a thickness of 150 nm to form the cathode 8. The organic EL device thus produced was measured for characteristics in the atmosphere at ordinary temperature.

The measurement results of the luminescence characteristics of the organic EL device produced by using the compound of Example 1 of the invention (Compound 8) upon application of a direct current voltage are shown in Table 1.

Comparative Example 1

For comparison, an organic EL device was produced under the same conditions as in Example 5 except that TPBI and FIrpic as a blue phosphorescent material were formed as the light emitting layer 5 in Example 5 in a thickness of 20 nm by the dual vapor deposition performed at a vapor deposition rate ratio of (TPBI)/(FIrpic) = 91/9. The organic EL device thus produced was measured for characteristics in the atmosphere at ordinary temperature.
The measurement results of the luminescence characteristics of the resulting organic EL device upon application of a direct current voltage are shown in Table 1.

Table 1

| | Compound | Voltage (V) (at 10 $mA/cm^2$) | Luminance ($cd/m^2$) (at 10 $mA/cm^2$) | Luminous efficiency (cd/A) (at 10 $mA/cm^2$) | Power efficiency (1m/W) (at 10 $mA/cm^2$) |
|---|---|---|---|---|---|
| Example 5 | Compound 8 | 6.57 | 2.365 | 23.65 | 11.30 |
| Comparative Example 1 | TPBI | 7.96 | 465 | 4.65 | 1.84 |

As shown in Table 1, the driving voltage under a current flown at a current density of 10 $mA/cm^2$ was 7.96 V for TPBI but was lowered to 6.57 V for the compound of Example 1 of the invention (Compound 8). The luminance, luminous efficiency and power efficiency under a current flown at a current density of 10 $mA/cm^2$ in the compound of Example 1 of the invention (Compound 8) were enhanced as compared to TPBI.

It is understood from the results above that the compound of the invention has a high triplet excitation level, favorably transmits energy to the phosphorescent material, and completely confines the triplet excitons of the phosphorescent material, and thus the compound is excellent as a host compound of a light emitting layer.

Industrial Applicability

The compound of the invention having a triazole ring structure to which a pyridyl group substituted with a carbazolyl group is bonded, has physical characteristics including (1) a high triplet excitation level, (2) a bipolar transporting property, and (3) stability in a thin film state, and may be used as a constitutional material of an electron transport layer, a hole blocking layer or a light emitting layer. The production of an organic EL device using the compound remarkably improves the luminance and the luminous efficiency of a conventional organic EL device, and thus can improve the

performance of mobile electronic products.

Explanation of Symbols

1 glass substrate
2 transparent electrode
3 hole transport layer
4 electron blocking layer
5 light emitting layer
6 electron transport layer
7 electron injection layer
8 cathode

## Claims

**1.** A compound of the following general formula (1) having a triazole ring structure to which a substituted pyridyl group is bonded,

[Chemical Formula 1]

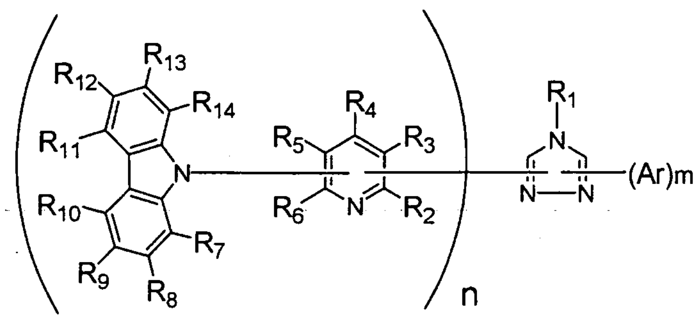

(1)

wherein Ar represents a substituted or unsubstituted aromatic hydrocarbon group, a substituted or unsubstituted aromatic heterocyclic group or a substituted or unsubstituted condensed polycyclic aromatic group; $R_1$ represents a substituted or unsubstituted aromatic hydrocarbon group, a substituted or unsubstituted aromatic heterocyclic group or a substituted or unsubstituted condensed polycyclic aromatic group; two of $R_2$, $R_3$, $R_4$, $R_5$ and $R_6$ represent a bonding group to a triazole ring or a carbazolyl group, and the others may be the same or different from each other, each representing a hydrogen atom, a deuterium atom, a fluorine atom, a cyano group, a linear or branched alkyl group having from 1 to 6 carbon atoms, a substituted or unsubstituted aromatic hydrocarbon group, a substituted or unsubstituted aromatic heterocyclic group or a substituted or unsubstituted condensed polycyclic aromatic group ; $R_7$, $R_8$, $R_9$, $R_{10}$, $R_{11}$, $R_{12}$, $R_{13}$ and $R_{14}$ may be the same or different from each other, each representing a hydrogen atom, a deuterium atom, a fluorine atom, a cyano group, a linear or branched alkyl group having from 1 to 6 carbon atoms, a substituted or unsubstituted aromatic hydrocarbon group, a substituted or unsubstituted aromatic heterocyclic group or a substituted or unsubstituted condensed polycyclic aromatic group; m represents 0 or 1; and n represents an integer of 1 or 2, provided that a sum of m and n is 2.

**2.** The compound having a triazole ring structure according to claim 1, wherein in the general formula (1), m = 1 and n = 1.

**3.** The compound having a triazole ring structure according to claim 1, wherein in the general formula (1), m = 0 and n = 2.

**4.** An organic electroluminescent device comprising a pair of electrodes and at least one organic layer sandwitched therebetween, at least one organic layer containing, as a constitutional material, a compound of the following general formula (1) having a triazole ring structure to which a substituted pyridyl group is bonded,

[Chemical Formula 2]

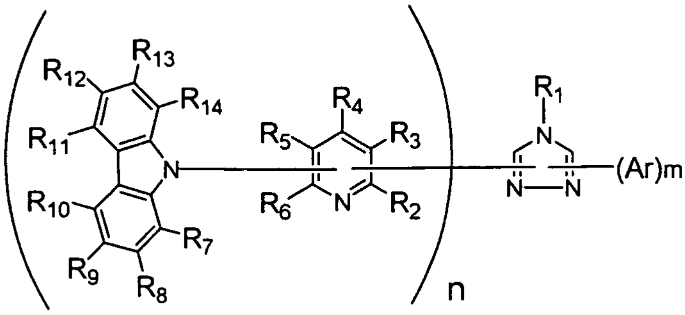

(1)

wherein Ar represents a substituted or unsubstituted aromatic hydrocarbon group, a substituted or unsubstituted aromatic heterocyclic group or a substituted or unsubstituted condensed polycyclic aromatic group; $R_1$ represents a substituted or unsubstituted aromatic hydrocarbon group, a substituted or unsubstituted aromatic heterocyclic group or a substituted or unsubstituted condensed polycyclic aromatic group; two of $R_2$, $R_3$, $R_4$, $R_5$ and $R_6$ represent a bonding group to a triazole ring or a carbazolyl group, and the others may be the same or different from each other, each representing a hydrogen atom, a deuterium atom, a fluorine atom, a cyano group, a linear or branched alkyl group having from 1 to 6 carbon atoms, a substituted or unsubstituted aromatic hydrocarbon group, a substituted or unsubstituted aromatic heterocyclic group or a substituted or unsubstituted condensed polycyclic aromatic group; $R_7$, $R_8$, $R_9$, $R_{10}$, $R_{11}$, $R_{12}$, $R_{13}$ and $R_{14}$ may be the same or different from each other, each representing a hydrogen atom, a deuterium atom, a fluorine atom, a cyano group, a linear or branched alkyl group having from 1 to 6 carbon atoms, a substituted or unsubstituted aromatic hydrocarbon group, a substituted or unsubstituted aromatic heterocyclic group or a substituted or unsubstituted condensed polycyclic aromatic group; m represents 0 or 1; and n represents an integer of 1 or 2, provided that a sum of m and n is 2.

5. The organic electroluminescent device according to claim 4, wherein in the general formula (1), m = 1 and n = 1.

6. The organic electroluminescent device according to claim 4, wherein in the general formula (1), m = 0 and n = 2.

7. The organic electroluminescent device according to claim 4, wherein the organic layer is an electron transport layer, and the electron transporting contains, as at least one constitutional material, the compound represented by the general formula (1).

8. The organic electroluminescent device according to claim 4, wherein the organic layer is a hole blocking layer, and the hole blocking layer contains, as at least one constitutional material, the compound represented by the general formula (1).

9. The organic electroluminescent device according to claim 4, wherein the organic layer is a light emitting layer, and the light emitting layer contains, as at least one constitutional material, the compound represented by the general formula (1).

FIG. 1

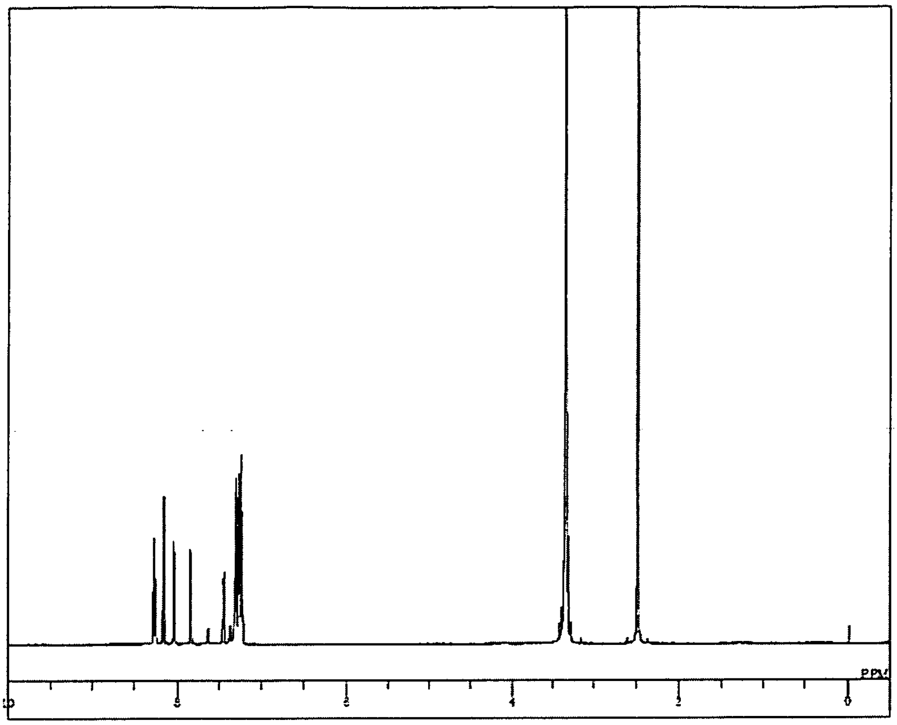
PPM

FIG. 2

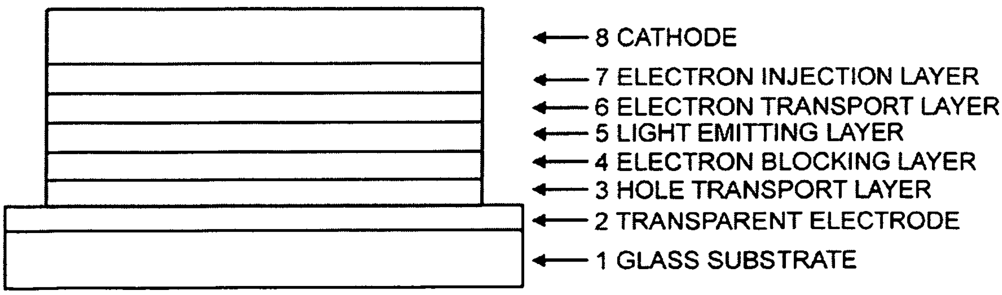
8 CATHODE
7 ELECTRON INJECTION LAYER
6 ELECTRON TRANSPORT LAYER
5 LIGHT EMITTING LAYER
4 ELECTRON BLOCKING LAYER
3 HOLE TRANSPORT LAYER
2 TRANSPARENT ELECTRODE
1 GLASS SUBSTRATE

**INTERNATIONAL SEARCH REPORT**

International application No.
PCT/JP2010/000243

A. CLASSIFICATION OF SUBJECT MATTER
*C07D401/14*(2006.01)i, *C09K11/06*(2006.01)i, *H01L51/50*(2006.01)i

According to International Patent Classification (IPC) or to both national classification and IPC

B. FIELDS SEARCHED

Minimum documentation searched (classification system followed by classification symbols)
C07D401/00-421/14, C09K11/06-11/07, H01L51/50-51/56

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2000-68059 A (Toyo Ink Manufacturing Co., Ltd.), 03 March 2000 (03.03.2000), claims 1 to 4; paragraphs [0011], [0031] to [0032]; table 1; examples 1 to 20 (Family: none) | 1-9 |
| X<br>A | JP 2008-308490 A (Semiconductor Energy Laboratory Co., Ltd.), 25 December 2008 (25.12.2008), claims 2, 10 to 15; paragraphs [0016], [0084], [0132], [0189]; examples 1 to 13 & WO 2008/143019 A1 & US 2008/0286607 A1 | 1-2,4-5,7-9<br>3,6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 February, 2010 (03.02.10) | 16 February, 2010 (16.02.10) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2010/000243

C (Continuation). DOCUMENTS CONSIDERED TO BE RELEVANT

| Category* | Citation of document, with indication, where appropriate, of the relevant passages | Relevant to claim No. |
|---|---|---|
| A | EP 1424381 A2 (KONICA MINOLTA HOLDINGS, INC.), 02 June 2004 (02.06.2004), & US 2004/0115476 A1 & JP 2004-311410 A & JP 2004-311412 A & JP 2004-311414 A & JP 2004-335427 A | 1-9 |
| A | KIM, J.H. et al, New host materials with high triplet energy level for blue-emitting electrophosphorescent device, Synthetic Metals, 2007, Vol.157, No.18-20, p.743-750 | 1-9 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007022986 A


### Non-patent literature cited in the description


- *Appl. Phys. Let.,* 1999, vol. 75, 4
- 9th Workshop, Molecular Electronics and Bioelectronics Group. *Japan Society of Applied Physics,* 2001, 17
- Yuki EL Display. Ohmsha, Ltd, 2005, 90
- *SID07 DIGEST,* 2007, 837
- *Journal of Molecular Electronics and Bioelectronics Group,* 2003, vol. 14 (1), 23
- Jikken Kagaku Koza. Maruzen Co., Ltd, 1992, vol. 7, 384-398
- *1st Meeting of Symposium for Organic EL,* 2005, 19